# EUROPEAN PATENT SPECIFICATION

(11) **EP 4 448 794 B1**
(45) Date of publication and mention of the grant of the patent: **21.01.2026**
(21) Application number: 22847698.2
(22) Date of filing: 14.12.2022
(51) Int. Cl.: C12Q 1/6851, C12Q 1/6888

(54) **COMPOSITIONS, KITS, AND METHODS FOR SHORT TANDEM REPEAT ANALYSIS**
ZUSAMMENSETZUNGEN, KITS UND VERFAHREN ZUR ANALYSE KURZER TANDEMWIEDERHOLUNGEN
COMPOSITIONS, TROUSSES ET PROCÉDÉS D'ANALYSE DE SÉQUENCES RÉPÉTÉES COURTES EN TANDEM

(30) Priority: 17.12.2021 US 202163291245 P; 17.12.2021 US 202163291271 P; 30.11.2022 US 202263428978 P
(43) Date of publication of application: 23.10.2024
(73) Proprietor: Life Technologies Corporation, Carlsbad, CA 92008 (US)
(72) Inventor: LAGACE, Robert, Carlsbad, CA 92008 (US)
(74) Representative: HGF
(86) International application number: PCT/US2022/081552
(87) International publication number: WO 2023/114841

(56) References cited:
- WO-A2-2011/066467
- US-A1- 2008 153 099
- GREEN ROBERT ET AL: "Developmental validation of VeriFiler(TM) Plus PCR Amplification Kit: A 6-dye multiplex assay designed for casework samples", FORENSIC SCIENCE INTERNATIONAL: GENETICS, ELSEVIER BV, NETHERLANDS, vol. 53, 13 March 2021 (2021-03-13), XP086616338, ISSN: 1872-4973, [retrieved on 20210313], DOI: 10.1016/J.FSIGEN.2021.102494
- LUDEMAN MATTHEW J ET AL: "Developmental validation of GlobalFiler(TM) PCR amplification kit: a 6-dye multiplex assay designed for amplification of casework samples", INTERNATIONAL JOURNAL OF LEGAL MEDICINE, SPRINGER BERLIN HEIDELBERG, BERLIN/HEIDELBERG, vol. 132, no. 6, 9 March 2018 (2018-03-09), pages 1555 - 1573, XP037215995, ISSN: 0937-9827, [retrieved on 20180309], DOI: 10.1007/S00414-018-1817-5
- KIM JEONGYONG ET AL: "Development and forensic validation of human genomic DNA quantification kit", INTERNATIONAL JOURNAL OF LEGAL MEDICINE, SPRINGER BERLIN HEIDELBERG, BERLIN/HEIDELBERG, vol. 134, no. 3, 15 August 2019 (2019-08-15), pages 963 - 975, XP037104673, ISSN: 0937-9827, [retrieved on 20190815], DOI: 10.1007/S00414-019-02131-Z
- SWANGO ET AL: "Developmental validation of a multiplex qPCR assay for assessing the quantity and quality of nuclear DNA in forensic samples", FORENSIC SCIENCE INTERNATIONAL, ELSEVIER B.V, AMSTERDAM, NL, vol. 170, no. 1, 30 June 2007 (2007-06-30), pages 35 - 45, XP022136537, ISSN: 0379-0738, DOI: 10.1016/J.FORSCIINT.2006.09.002
- HOLT ALLISON ET AL: "Developmental validation of the Quantifiler HP and Trio Kits for human DNA quantification in forensic samples", FORENSIC SCIENCE INTERNATIONAL: GENETICS, ELSEVIER BV, NETHERLANDS, vol. 21, 29 December 2015 (2015-12-29), pages 145 - 157, XP029412006, ISSN: 1872-4973, DOI: 10.1016/J.FSIGEN.2015.12.007
- JON WILSON ET AL: "Molecular Assay for Screening and Quantifying DNA in Biological Evidence: The Modified Q-TAT Assay*", JOURNAL OF FORENSIC SCIENCES, vol. 55, no. 4, 25 March 2010 (2010-03-25), pages 1050 - 1057, XP055012983, ISSN: 0022-1198, DOI: 10.1111/j.1556-4029.2010.01371.x

## Description

This application claims the benefit of and priority to United States Provisional Patent Application Numbers: 63/291,245 filed December 17, 2021; 63/291,271 filed December 17, 2021; and 63/428,978 filed November 30, 2022.

### BACKGROUND

This disclosure relates to compositions, kits, and methods for use in applications involving electrophoretic separation of nucleic acids, including capillary electrophoresis applications in which nucleic acid samples are labelled with dyes, size-separated, and subjected to short tandem repeat (STR) analysis.

Rapid DNA testing is typically understood to mean a process that generates a DNA profile from a sample in a matter of hours rather than days and that requires limited human intervention. For example, law enforcement can obtain a cheek swab of an individual under arrest and insert the sample into a rapid DNA testing instrument. At present, such instruments provide a DNA profile within about two hours. The DNA profile may then be compared to DNA profile databases and/or to DNA samples associated with unsolved crimes. Rapid DNA testing is also utilized in forensic analysis, identification of human remains, crime scene investigations, and the identification or exclusion of suspects at the booking station.

Rapid DNA instruments typically utilize electrophoresis to separate DNA within a sample based on size, and to provide a DNA profile based on STR typing. STR loci are targeted with specified primers and are then amplified and labelled with dyes. Often, multiple dyes are used, each dye being specific to a particular locus or set of loci that are expected to sufficiently spread out once size separated. This allows for allele typing of multiple loci spread among the different dye channels. With enough loci analyzed, the pattern of alleles provides highly accurate identification of an individual. In the U.S., for example, STR typing procedures commonly analyze the standard core loci of the Combined DNA Index System (CODIS), referred to as the CODIS 20 (or the core loci of the previous CODIS 13 standard).

Rapid DNA applications are beneficial due to the relative speed in which they provide results. Results can be obtained while suspects are still in custody, reducing the risk that the suspect will flee and be unreachable after DNA results are obtained. Rapid DNA applications also reduce the number of processing steps, the number of people handling the sample, and the number of location transfers required to obtain results. This lowers the risk of sample contamination or mishandling.

However, rapid DNA applications also have several limitations. DNA samples obtained from crime scenes, for example, often contain environmental debris and may include mixed DNA from multiple individuals. Moreover, because the amount of collected DNA varies depending on the amount available at the scene, the method of obtaining the sample, the type of sample (e.g., blood vs. touch/trace DNA), and the skill of the person(s) collecting the sample, the results can often be noisy compared to a full laboratory analysis.

Further, variation in DNA profile peak heights resulting from the above factors and/or from the random variation associated with amplification of the DNA (i.e., "stochastic effects") can hamper result quality. Peak height thresholds aim to manage the noise in the profile by only considering peaks above the threshold as representative of alleles. Higher thresholds can therefore eliminate more artifacts resulting from stochastic effects. However, DNA peaks below the threshold often represent real alleles, so a higher threshold also lowers the sensitivity of the analysis.

Ambiguities in the DNA profile resulting from such stochastic effects are exacerbated when the amount of DNA in the sample is low. However, conventional rapid DNA applications do not provide a means for quantitating the amount of DNA in the sample. Thus, a user may not know whether the level of DNA in a given sample is too low, or at what point it should be considered as too low, without additional testing and/or consultation with an expert.

In addition, rapid DNA testing may involve degraded DNA and/or inhibited amplification of DNA. The resulting DNA profiles may have low quality and/or may require interpretation by an expert to resolve issues and ambiguities. A typical booking station is unlikely to have such experts on hand and readily available. Where expert interpretation is required, the application becomes much less "rapid", and the potential benefits of rapid testing are not realized.

US 2008/153099 A1 (ALLEN ROBERT W [US] ET AL; 26 June 2008) discloses a kit and assay for DNA profiling in forensic settings based on multiplex PCR amplification of STR markers: VeriFiler Plus. The method involves simultaneous amplification of: - 23 autosomal STR loci; - 2 internal quality control markers: IQCS and IQCL; - 2 gender discrimination markers: Y-indel and amelogenin. The IQCS and IQCL primer sets are designed to amplify two exogenous, synthetic DNA sequences that help distinguish DNA sample degradation from amplification reaction inhibition. In addition, the IQC system serves as a positive control for PCR amplification. Further, said method involves a DNA quantitation step that is carried out before the STR multiplex amplification reaction and involves quantitative PCR amplification of standard targets: Quantifiler System. The Quantifiler system is described by HOLT ALLISON ET AL, 2015 (FORENSIC SCIENCE INTERNATIONAL: GENETICS, vol. 21, pages 145-157) and comprises primers for multiplex qPCR of two different multicopy autosomal targets, small (80 bp) and large (240 bp), which allows an assessment of sampleDNA quantity and quality (e.g. degradation) prior to the SRT profiling as such.

Accordingly, there is an ongoing need for improvements in rapid DNA testing applications. The present application is directed to compositions, kits, and methods that represent, in at least some implementations, an improvement over conventional rapid DNA testing.

### BRIEF DESCRIPTION OF THE DRAWINGS

Various objects, features, characteristics, and advantages of the invention will become apparent and more readily appreciated from the following description of the embodiments, taken in conjunction with the accompanying drawings and the appended claims, all of which form a part of this specification. In the Drawings, like reference numerals may be utilized to designate corresponding or similar parts in the various Figures, and the various elements depicted are not necessarily drawn to scale, wherein:
Figure 1A illustrates an example capillary electrophoresis instrument as an example of one application in which the present compositions, kits, and methods may be utilized;
Figure 1B illustrates an example STR electropherogram;
Figures 2A and 2B list example QS sequences and corresponding QS primers and example QL sequences and corresponding QL primers, respectively;
Figure 3A illustrates an example STR electropherogram that includes a short quantification standard (QS standard), a long quantification standard (QL standard), a short internal quality control (IQCS), and a long internal quality control (IQCL);
Figure 3B illustrates an example STR electropherogram resulting from use of the QS standard, QL standard, IQCS, and IQCL, the results indicating that the level of genomic DNA in the sample is relatively low;
Figure 3C illustrates an example STR electropherogram resulting from use of the QS standard, QL standard, IQCS, and IQCL, the results indicating negative detection of genomic DNA in the sample;
Figure 3D illustrates an example STR electropherogram resulting from use of the QS standard, QL standard, IQCS, and IQCL, the results indicating that genomic DNA in the sample is degraded;
Figure 3E illustrates an example STR electropherogram resulting from use of the QS standard, QL standard, IQCS, and IQCL, the results indicating inhibited amplification of genomic DNA in the sample;
Figure 4 is a multi-channel electropherogram showing results using QS and QL markers selected from those shown in Figures 2A and 2B;
Figure 5A is a chart illustrating peak heights of STR profiles resulting from a blood dilution series that varied the volume of blood utilized in the rapid DNA analysis, the results showing that suitable peak heights were obtained at blood volumes as low as about 0.4 µL;
Figure 5B is a chart illustrating peak height ratios for different blood volumes at various tested loci, the results showing suitable performance at blood volumes as low as about 0.4 µL;
Figure 6A is a table comparing results of a conventional rapid DNA testing process to an improved process according to the present disclosure, the improved process being modified to use a different collection swab, a lower lysate volume, and lower number of PCR cycles, showing that the modified process reduced the number of flags while also providing effective peak heights;
Figures 6B and 6C are charts illustrating peak heights (Figure 6B) and peak height ratios (Figure 6C) at various loci, comparing a conventional rapid DNA testing process ("RI Cotton 1 µL blood") to an improved process according to the present disclosure ("BH1 HydraFlock 1 µL blood"), the results showing that the improved process led to less variance in peak heights;
Figure 7 illustrates results of DNA input level testing, showing changes in the QS standard and QL standard in the corresponding STR profiles at different levels of input DNA;
Figures 8A and 8B illustrate average peak heights for QS and QL sequences across different levels of gDNA input based on control DNA and liquid blood; and
Figure 9 illustrates results of DNA degradation testing, showing greater changes to the QL standard than the QS standard in the corresponding STR profiles at progressively higher levels of DNA degradation.

### DETAILED DESCRIPTION

### Example Analysis Instrument

The present disclosure includes specific examples directed to rapid DNA analysis systems using capillary electrophoresis. However, the skilled person will understand, in light of this disclosure, that the compositions, kits, and methods described herein may be additionally or alternatively utilized in other applications such as traditional gel electrophoresis applications. In addition, although several examples are described in the context of rapid DNA analysis, it will be understood that the embodiments described herein may also be beneficially utilized in other nucleic acid analysis applications, including conventional laboratory analysis applications.

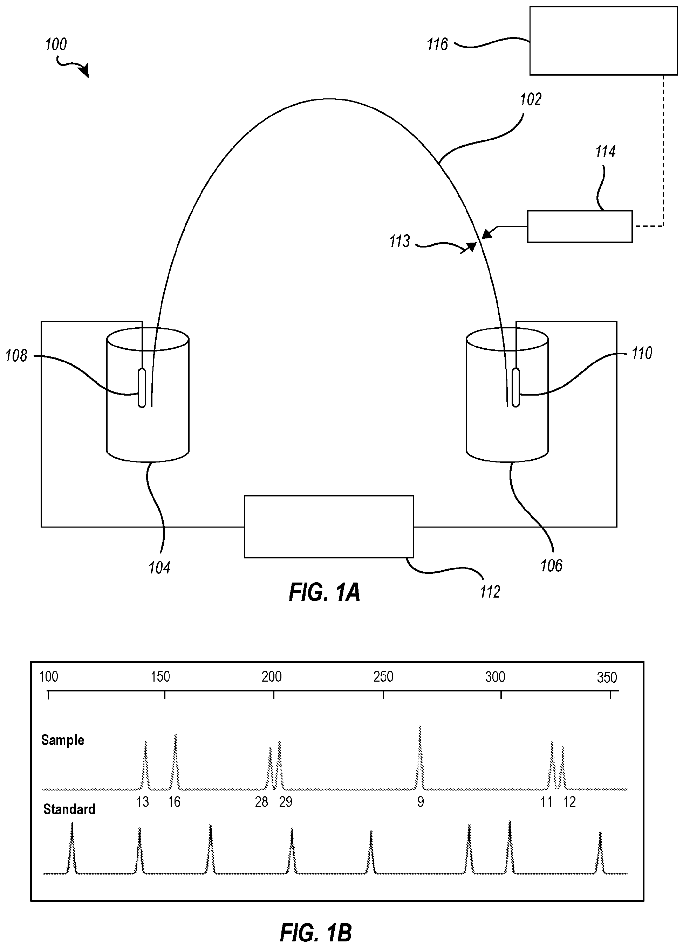

Figure 1A schematically illustrates a capillary electrophoresis instrument 100 as one example of an application in which the presently described embodiments may be implemented. The capillary electrophoresis instrument 100 includes a capillary 102 with one end in contact with a source container 104 and the opposite end in contact with a destination container 106. The capillary 102 is filled with a sieving polymer. Typically, multiple capillaries are included in a capillary electrophoresis system to allow for parallel analysis of multiple samples, though for the sake of clarity only one capillary 102 is illustrated here. The use of capillaries, as opposed to conventional slab gels, allows for the use of stronger electrical fields, which results in faster separation and increased overall throughput.

The source container 104 and destination container 106 hold an appropriate electrolytic buffer, and the sample to be analyzed is added to the source container 104 or otherwise mixed with the buffer at the source container 104 when introduced into the capillary 102.

The capillary electrophoresis instrument 100 also includes a pair of electrodes 108 and 110 that are in electrical communication with the buffer solution at the opposing containers 104 and 106. A power supply 112 generates a voltage between the electrodes 108 and 110. The sample is introduced into the capillary 102 and the electric potential between electrodes 108 and 110 then causes the targeted analytes to migrate through the capillary 102 toward the destination container 106.

The analytes (e.g., nucleic acids) separate by size as they migrate through the capillary 102 according to differences in electrophoretic mobility through the capillary. That is, smaller fragments will move through the polymer faster than larger fragments. In applications where nucleic acids are the analytical target (as in this example), the negatively charged nucleic acid fragments will move from the negatively charged cathode 108 toward the positively charged anode 110 under the applied voltage.

The capillary 102 includes a detection window 113 coincident with a corresponding detector assembly 114. The detector assembly includes a laser and a fluorescence detector. As dye-labelled nucleic acid fragments pass through the detection window, the laser excites the dye-labelled fragments and the detector (e.g., a charge-coupled device (CCD) camera) detects the resulting fluorescence signals. Typically, multiple different dyes that each provide a different, known fluorescence response to the excitation light are used to label different nucleic acid targets. Thus, the identities of the fragments may be determined according to the character of the corresponding fluorescence signal.

The detector assembly 114 is communicatively coupled to a computer device 116. The computer device 116 includes one or more processors and memory (e.g., one or more hardware storage devices) that enable it to receive the fluorescence signal data from the detector assembly 114 and to generate an electropherogram showing the detected fluorescence signals of the different dye "channels" over time. Peaks in the electropherogram indicate times at which a labelled fragment passed through the detection window 113. By comparing these detected peaks to standard peaks (i.e., a "ladder") from a sample having fragments of known size, the sizes of the fragments can be determined.

One major use of capillary electrophoresis is STR typing. STR loci are targeted with specified primers and are then amplified and labelled with dyes. Often, multiple dyes are used, each dye being specific to a particular locus or set of loci that are expected to sufficiently spread out once size separated. This allows for allele typing of multiple loci spread among the different dye channels. With enough loci analyzed, the pattern of alleles provides highly accurate identification of an individual. In the U.S., for example, STR typing procedures commonly analyze the standard core loci of the Combined DNA Index System (CODIS), referred to as the CODIS 20 (or the core loci of the previous CODIS 13 standard).

One example of a rapid DNA instrument that may be utilized with the disclosed compositions, kits, and methods is the RapidHIT ID System (Applied Biosystems, Catalog No. A41810). The RapidHIT ID System utilizes RapidINTEL^{™} sample cartridges (Applied Biosystems, Catalog No. A43942). The embodiments described herein may utilize similar components, dyes, STR target loci, and the like, but with the modifications described herein.

Figure 1B is an exemplary STR electropherogram. Common components of such an electropherogram include a size standard showing peaks from nucleic acid fragments of known size, the peaks of the test sample in one or more dye channels (a single channel shown here), and a size indicator showing the corresponding length (in number of base pairs) of the fragments. As shown, the peaks of the test sample are usually also annotated with a number to indicate the STR allele(s) detected at the particular locus associated with each peak or pair of peaks. Different alleles for a given STR locus have different sizes due to different numbers of the repeat sequence present. Loci labels are not shown here but are often also included.

### Quantification Standards

Certain embodiments described herein utilize quantification standards to improve nucleic acid analysis, including STR analysis included as part of a rapid DNA testing process. The QS and QL standards are designed to target regions of the genomic DNA of the sample, and do not include synthetic and/or additionally added target sequences. The QS standard includes a set of short quantification primers (QS primers) that target a first sequence (QS sequence) of the sample nucleic acid, whereas the QL standard includes a set of long quantification primers (QL primers) that target a second sequence (QL sequence) of the sample nucleic acid. The QS sequence is shorter than the QL sequence.

The QS and QL primers are used with at least one set of primers that target an STR locus of the sample nucleic acid. In some embodiments, a composition includes the QS and QL primers and multiple sets of primers each configured to target a different STR region of the sample nucleic acid. Examples of suitable STR regions are known in the art, including those targeted with the GlobalFiler^{™} IQC PCR amplification kit (Applied Biosystems, Catalog No. A43565) and/or other STR loci commonly analyzed in the forensics, criminal investigation, and human identification fields.

In some embodiments, the QS sequence is shorter than the targeted STR regions and the QL sequence is longer than the targeted STR regions such that the QS standard and the QL standard bracket the targeted STR regions along the spectrum of analyzed sizes. However, in other embodiments, the QS and/or the QL sequence(s) have sizes that fall within the size range of the STR regions. In such embodiments, the QS and/or QL marker(s) preferably have their own unique size range and/or unique dye label to be sufficiently distinguished from the targeted, STR loci on the electropherogram.

The QS and/or QL sequences may be sequences that have a single copy within the human genome. In other embodiments, the QS and/or QL sequences are multi-copy sequences within the human genome. The QS and QL sequences are also preferably free of indels to prevent size variation across samples and/or across different regions of the multi-copy sequence.

In some embodiments, the QS sequence has a size ranging from about 40 to about 120 nucleotides, or about 50 to about 110 nucleotides, or about 60 to about 100 nucleotides, or about 70 to about 90 nucleotides, or a size within a range having endpoints defined by any two of the foregoing values. In some embodiments, the QL sequence has a size ranging from about 300 to about 600 nucleotides, or about 325 to about 575 nucleotides, or about 350 to about 550 nucleotides, or about 375 to about 525 nucleotides, or about 400 to about 500 nucleotides, or about 420 to about 480 nucleotides, or about 440 to about 460 nucleotides, or a size within a range having endpoints defined by any two of the foregoing values.

The sizes of the QS and QL sequences may be varied according to particular application needs, and need not fall within the ranges disclosed above. For example, depending on the particular STR regions targeted and the range of potential allele sizes for such targeted STR loci, the QS and QL sizes can be adjusted so as to bracket the targeted STR loci. Moreover, as discussed above, in certain embodiments the QS and/or QL sequence(s) have sizes that fall within the size range of the targeted STR regions, and the QS and QL sequences need not necessarily completely bracket all targeted STR loci.

In some embodiments, the set of QS primers includes a forward primer and a reverse primer to target the corresponding QS sequence. At least some of the forward primer molecules and/or at least some of the reverse primer molecules of the set of QS primers include a detectable label. In some embodiments, not all of the forward primer molecules and/or reverse primer molecules are labelled. Limiting the number of labelled molecules beneficially provides more control over the expected peak height of the QS standard.

For example, the ratio of labelled to non-labelled primers can be controlled to provide an effective peak height (e.g., not excessively short or excessively tall) while still providing sufficient overall levels of primers to drive effective amplification of the QS sequence. In some embodiments, the ratio of labelled to non-labelled forward primers in the set of QS primers is about 1:1 to about 1:5, such as about 1:1, 1:2, 1:2, 1:4, 1:5, or a range of ratios with any of the foregoing as endpoints, and/or the ratio of labelled to non-labelled reverse primers in the set of QS primers is about 1:1 to about 1:5, such as about 1:1, 1:2, 1:2, 1:4, 1:5, or a range of ratios with any of the foregoing as endpoints.

In some embodiments, the set of QL primers includes a forward primer and a reverse primer to target the corresponding QL sequence. At least some of the forward primer molecules and/or at least some of the reverse primer molecules of the set of QL primers include a detectable label. In some embodiments, not all of the forward primer molecules and/or reverse primer molecules are labelled. Limiting the number of labelled molecules beneficially provides more control over the expected peak height of the QL standard.

For example, the ratio of labelled to non-labelled primers can be controlled to provide an effective peak height (e.g., not excessively short or excessively tall) while still providing sufficient overall levels of primers to drive effective amplification of the QL sequence. In some embodiments, the ratio of labelled to non-labelled forward primers in the set of QL primers is about 1:1 to about 1:5, such as about 1:1, 1:2, 1:2, 1:4, 1:5, or a range with any of the foregoing ratios as endpoints, and/or the ratio of labelled to non-labelled reverse primers in the set of QL primers is about 1:1 to about 1:5, such as about 1:1, 1:2, 1:2, 1:4, 1:5, or a range with any of the foregoing ratios as endpoints.

The QS primers and/or QL primers described herein need not have 100% homology to their respective QS sequence and QL sequence targets to be effective, though in some embodiments, homology is substantially 100%. In some embodiments, one or more of the disclosed primers have a homology to their respective target of about 70%, about 80%, about 85%, about 90%, about 95%, about 97%, about 98%, about 99%, about 99.5%, up to substantially 100%, or a range with endpoints defined by any two of the foregoing percentage values. Some combinations of primers may include primers each with different homologies to their respective targets, and the homologies may be, for example, within a range with endpoints defined by any two of the foregoing percentage values.

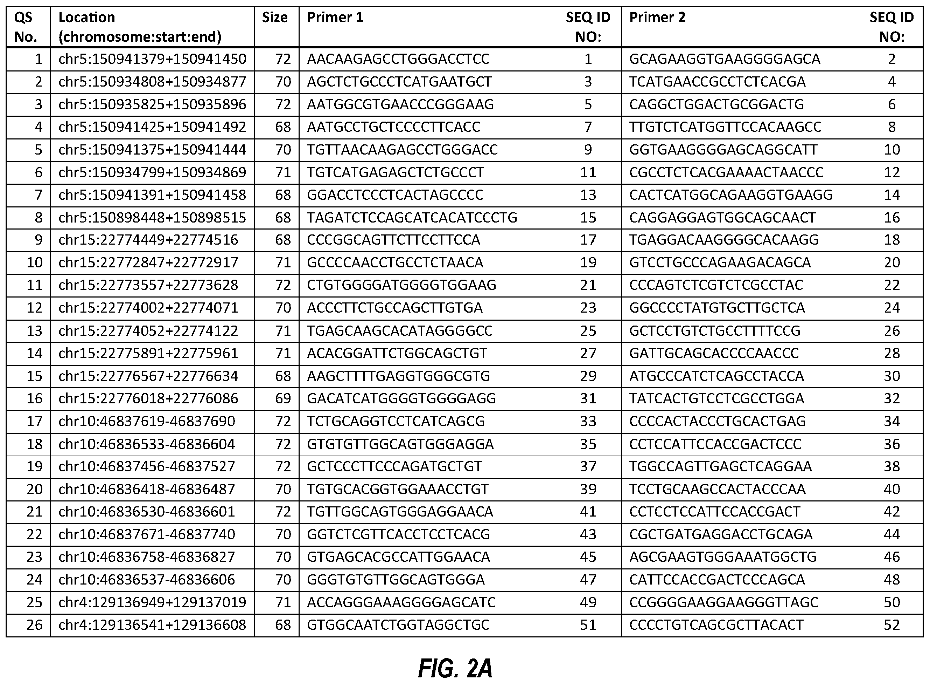

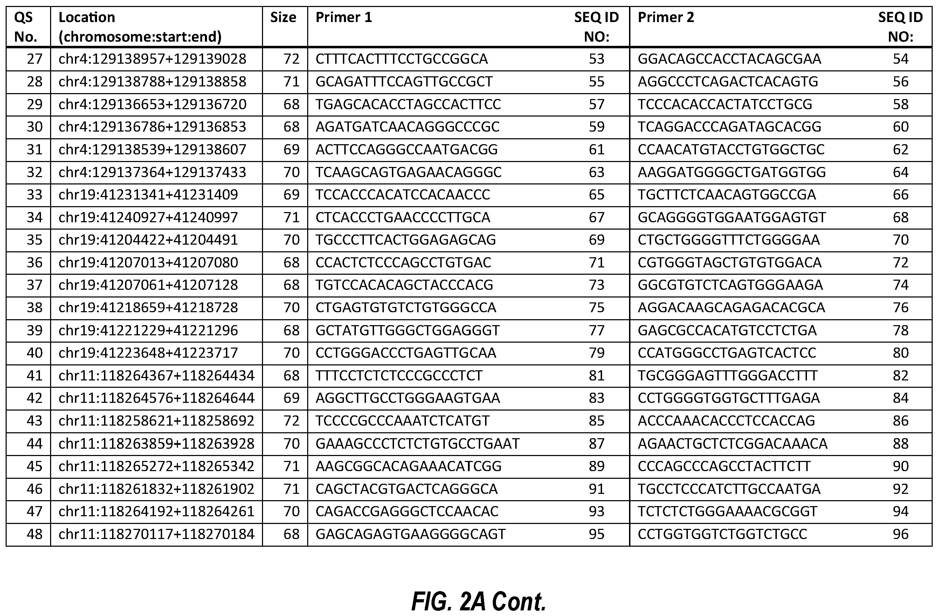

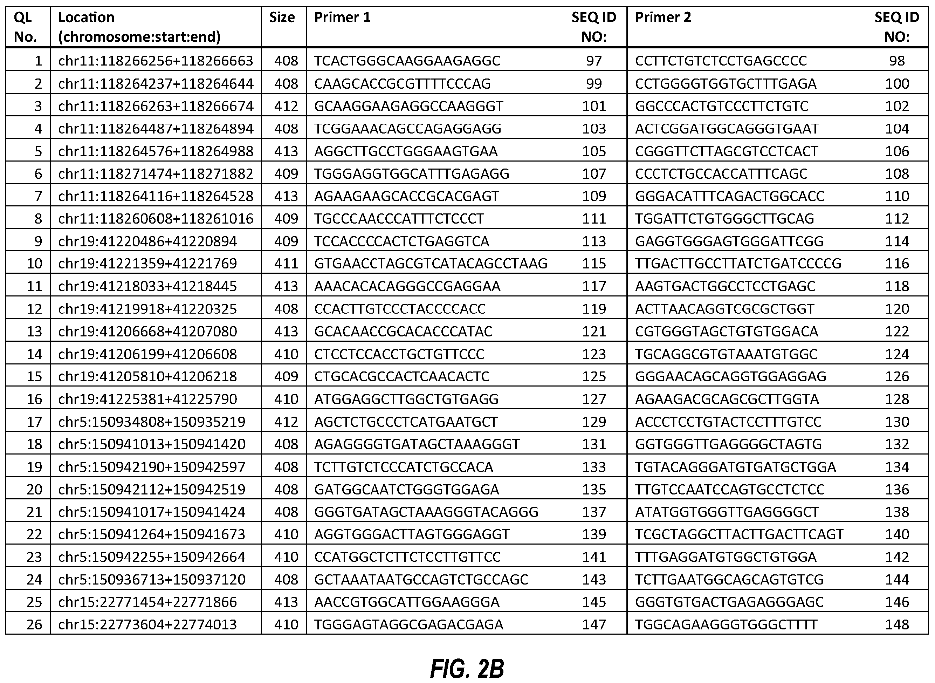

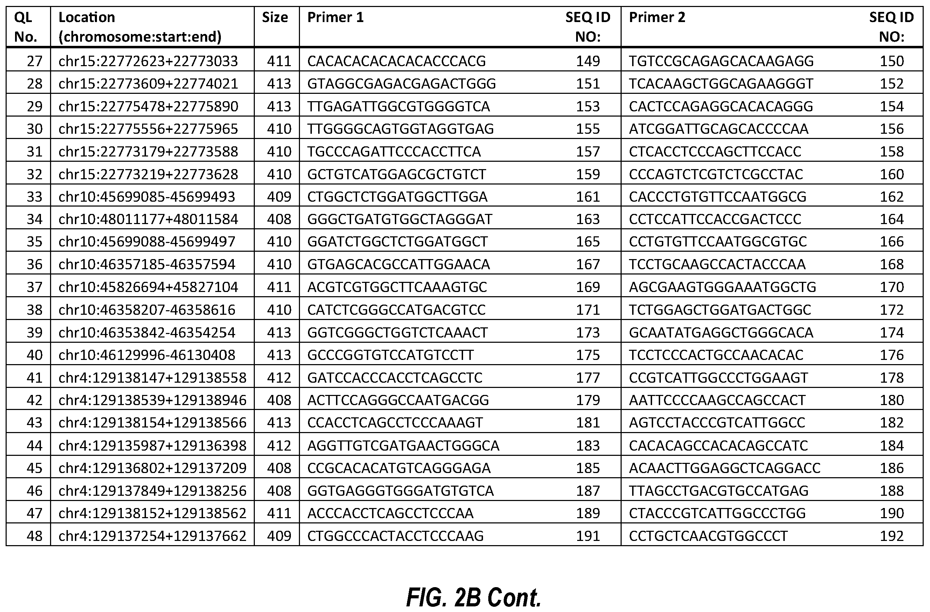

Examples of QS sequences and corresponding QS primers are listed in the table of Figure 2A. Examples of QL sequences and corresponding QL primers are listed in the table of Figure 2B. In these Figures, the start and end human genome locations are based on the GenBank Genome Reference Consortium Human Reference 38 (GRCh38; hg38).

### Compositions & Kits for DNA Amplification and Analysis

In some embodiments, the QS and QL standards are included as part of a composition and/or kit for amplifying and analyzing DNA samples. In some embodiments, for example, the composition includes (i) a set of QS primers, (ii) a set of QL primers, (iii) one or more sets of primers each targeted to an STR region of the sample nucleic acid, (iv) optionally, a short internal quality control (IQCS) comprising a synthetic IQCS sequence and a set of primers configured to enable amplification of the IQCS sequence, and (v) optionally, a long internal quality control (IQCL) comprising a synthetic IQCL sequence and a set of primers configured to enable amplification of the IQCL sequence. In some embodiments, the IQCS sequence is shorter than the targeted STR region(s) and the IQCL sequence is longer than the targeted STR region(s). In such embodiments, the IQCS and IQCL bracket the targeted STR regions along the spectrum of analyzed sizes. In other embodiments, the IQCS and/or IQCL sequence(s) have sizes that fall within the range of targeted, variably sized STR loci. In such embodiments, the IQCS and/or IQCL preferably have their own unique size range and/or unique dye label to be sufficiently distinguished from the targeted STR loci on the electropherogram.

The IQCS and IQCL are configured as internal process controls. That is, the IQCS and IQCL can provide information regarding the quality of the testing process, such as whether amplification has been inhibited. However, because the IQCS and IQCL are amplified from their own synthetic template, they cannot function as quantitative standards and cannot provide information specific to the genomic DNA of the sample.

In some embodiments, a kit configured for analysis of nucleic acid samples includes (i) a set of QS primers, (ii) a set of QL primers, (iii) one or more sets of primers each targeted to an STR region of the sample nucleic acid, (iv) optionally, an IQCS, (v) optionally, an IQCL, and (vi) one or more components to enable amplification of target DNA when the kit components are mixed with a sample and subjected to amplification conditions (e.g., thermal cycling to perform polymerase chain reaction (PCR)). The one or more components for enabling amplification may include a lysate, a DNA polymerase, deoxyribonucleotide triphosphate (dNTP) molecules, and one or more buffers and/or salts. In some embodiments, the DNA polymerase is a thermostable DNA polymerase, such as Taq DNA polymerase, a mutant, variant, or derivative thereof. In some embodiments, the dNTP molecules are selected from the group consisting of dTTP, dATP, dCTP, dGTP, 7-deaza-dGTP, or dUTP. Additional or alternative components for enabling amplification of target DNA are known in the art.

### Methods of Amplifying Nucleic Acids & Identifying Individuals

In some embodiments, a method of amplifying one or more nucleic acids within a sample comprises the steps of: (A) providing a sample that includes or is suspected of including nucleic acid; (B) forming a mixture by mixing with the sample a composition comprising (i) at least one set of primers that target an STR region of the sample nucleic acid, (ii) a set of QS primers that target a first QS sequence of the sample nucleic acid, and (iii) a set of QL primers that target a second sequence of the sample nucleic acid, wherein the QS sequence is shorter than the QL sequence; and (C) subjecting the mixture to amplification conditions to enable amplification of targeted nucleic acid if present within the sample.

In some embodiments, the method further comprises identifying an allele of the STR region(s). In some embodiments, the method further comprises identifying an individual based on the determined allele(s).

In some embodiments, a method for identifying a human by analyzing a sample containing nucleic acid from the human comprises the steps of: (A) providing a sample that includes or is suspected as including nucleic acid from the human; (B) forming a mixture by mixing with the sample a composition comprising (i) multiple sets of primers each configured to target a different STR region of the sample nucleic acid, (ii) a set of QS primers that target a QS sequence of the sample nucleic acid, and (iii) a set of QL primers that target a QL sequence of the sample nucleic acid, wherein the QS sequence is shorter than the QL sequence; (C) subjecting the mixture to amplification conditions to enable amplification of targeted nucleic acid within the sample; (D) identifying the alleles of the different STR regions; and (E) identifying the human based on the determined alleles.

In some embodiments, the sample includes or is suspected as including human genomic DNA. In some embodiments, the sample is a forensic sample, crime scene sample, victim sample, or sample from unidentified human remains. The sample may include, for example, blood, bone, buccal material, saliva, semen, urine, feces, skin cells, touch DNA, or a combination thereof.

As discussed above, one limitation common to rapid DNA testing applications is excessive stochastic effects which require the raising of peak height thresholds to compensate. Reducing the number of PCR cycles reduces the number of artifacts present in the resulting STR profile, but also concomitantly reduces the sensitivity of the test. In situations where low levels of sample are available and low levels of DNA are expected (e.g., touch samples, dried blood or other bodily fluid traces on hard surfaces, clothing), the number of PCR cycles is preferably reduced. To compensate for the loss of sensitivity, such methods preferably also utilize flocked swabs for sample collection and/or reduce the relative volume of lysate.

In some embodiments, the sample is collected using a swab. The swab may be a standard cotton swab. Alternatively, in certain embodiments, the swab is a flocked swab. A flocked swab comprises an arrangement of multi-length synthetic fibers attached (e.g., via adhesive) to a substrate/surface. Flocked swabs are preferred in situations where high sensitivity is needed and/or where low levels of sample are available and low levels of DNA are expected. Where larger amounts of sample are available (e.g., buccal swab or sufficient blood), a standard, cotton-tipped swab is acceptable. The volume of sample used as input for amplification is preferably at least about 0.1 µL (e.g., 0.075 µL or greater), or at least about 0.2 µL, or at least about 0.3 µL, or at least about 0.4 µL, or at least about 0.5 µL. The volume of sample used as input is also preferably no greater than about 30 µL, or no greater than about 35 µL, or no greater than about 40 µL. In preferred embodiments, a swab or other sample collection device is configured to absorb and "hold" no more than about 40 µL of the sample and/or no more than about 50% of the available sample.

In some embodiments, the sample is mixed with a lysate before and/or at initiation of amplification. The lysate volume may be varied according to protocol and/or sample amount. In some embodiments, the lysate volume is about 300 µL or more, such as about 500 µL. Alternatively, in certain embodiments, the lysate volume is less than about 300 µL, or less than about 250 µL, or less than about 200 µL, or less than about 150 µL, or about 100 µL. For example, the composition with which the sample is mixed may comprise the lysate at any of the foregoing volumes. Lower lysate volumes are preferred in situations where high sensitivity is needed and/or where low levels of sample are available and low levels of DNA are expected. Where larger amounts of sample are available, the larger lysate volumes (e.g., 300 µL or more) are acceptable.

In some embodiments, the sample is subjected to about 32 cycles of PCR. Alternatively, in certain embodiments, the sample is subjected to less than 32 cycles of PCR, such as 30 cycles. Lower cycle numbers are preferred in situations where high sensitivity is needed and/or where low levels of sample are available and low levels of DNA are expected. Where larger amounts of sample are available, higher cycle numbers (e.g., 32 cycles) are acceptable. In some embodiments, the sample is subjected to more than 32 cycles of PCR. Such embodiments may be implemented where greater sensitivity is desired and results do not suffer from excessive stochastic effects, for example.

### Use of QS and QL Standards to Characterize DNA Profiles

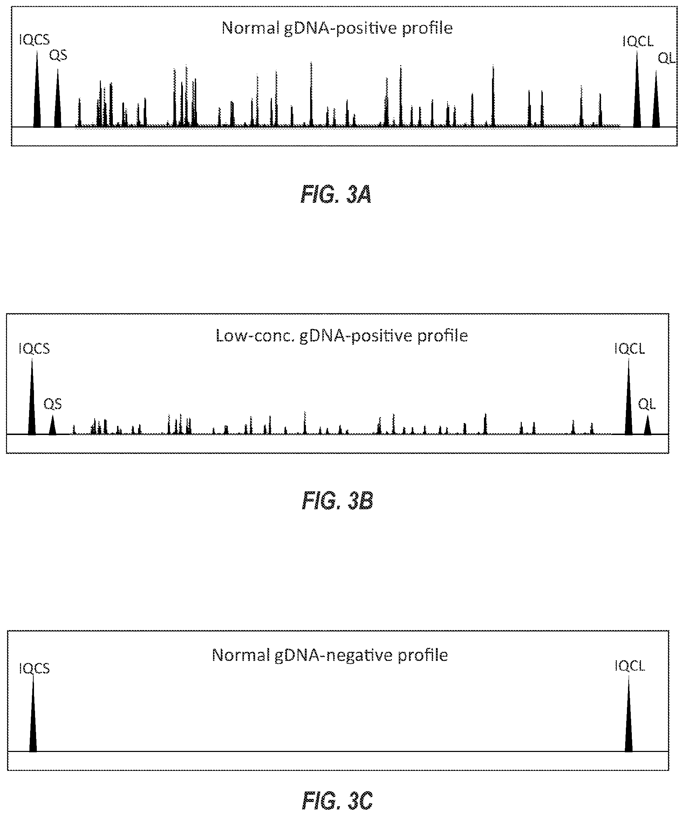

Figures 3A-3E illustrate example STR profiles (i.e., electropherograms) for different sample and/or testing scenarios. The STR profiles illustrated here include the QS standard, QL standard, IQCS, and IQCL. In some embodiments, the IQCS and/or IQCL may be omitted.

Figure 3A illustrates a normal STR profile. As shown, the QS and QL standards are visible and bracket the ends of the STR peaks. This example also includes visible IQCS and IQCL peaks bracketing the ends of the STR peaks. The presence of the IQCS and IQCL peaks indicates that the STR process (including the DNA amplification steps) were carried out effectively. The QS and QL peaks are also substantially similar in height, indicating that the shorter and longer DNA fragments of the sample were similarly amplified.

Figure 3B illustrates an example STR electropherogram resulting from use of the QS standard, QL standard, IQCS, and IQCL, the results indicating that the level of genomic DNA in the sample is relatively low. As shown, the STR, QS, QL, IQCS, and IQCL peaks are all present, as with the normal profile of Figure 3A. However, the QS, QL, and STR peaks are lower relative to the profile of Figure 3A, whereas the IQCS and IQCL peaks remain similar to those of the profile of Figure 3A. This is because the IQCS and IQCL standards are process controls that utilize their own synthetic templates rather than the genomic DNA of the sample. The size of the IQCS and IQCL peaks therefore does not provide any information about the relative amounts of genomic DNA in the sample. On the other hand, the QS and QL standards are based on primers that target short and long, respectively, regions in the human genome. The QS and QL peaks are therefore proportional to the amount of genomic DNA in the sample and thus to the STR peaks as well.

Peak heights of the QS and/or QL standards can therefore be utilized to quantify the amount of genomic DNA in the sample using, for example, pre-generated standard curves. Such quantification may be utilized to provide a numerical estimate of genomic DNA and/or a categorization of DNA amount such as "high," "medium," and "low," or "stochastic" vs. "non-stochastic." Typically, the QS is better suited for quantification determinations whereas the QL (and/or a QS to QL ratio) is useful for determining whether the longer STR regions are associated with excessive degradation and/or inhibition effects.

Figure 3C illustrates an example STR electropherogram resulting from use of the QS standard, QL standard, IQCS, and IQCL, the results indicating negative detection of genomic DNA in the sample. As shown, although the IQCS and IQCL peaks are present, the STR, QS, and QL peaks are absent. The presence of the IQCS and IQCL peaks indicates that the synthetic IQCS and IQCL templates were properly amplified, and thus rules out any overall amplification errors. Had targeted genomic DNA been present in the sample, it would have been expected to result in corresponding QS, QL, and STR peaks. The absence of such peaks therefore indicates that the sample did not include targeted genomic DNA.

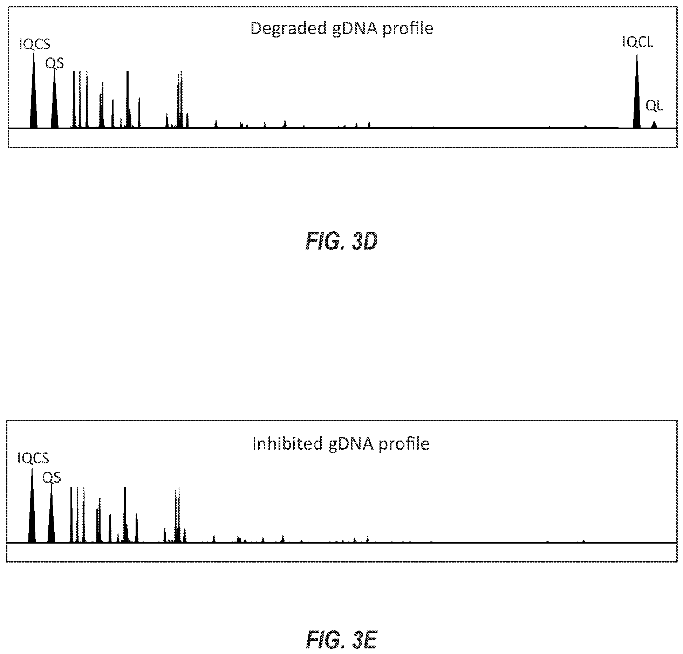

Figure 3D illustrates an example STR electropherogram resulting from use of the QS standard, QL standard, IQCS, and IQCL, the results indicating that genomic DNA in the sample is degraded. As shown, although some STR peaks are present, the peaks show a "ski-slope" profile in which the shorter loci tend to have higher peaks and longer loci have progressively shorter peaks. The IQCS and IQCL are both present. The QS is also present, but the QL is absent or has a height substantially reduced relative to the QS height. This type of profile indicates degraded DNA because longer DNA strands are more likely to be cleaved or otherwise degraded. The IQCL, however, maintains a height similar to the IQCS because it is based on a synthetic template separate from the degraded genomic DNA of the sample.

Figure 3E illustrates an example STR electropherogram resulting from use of the QS standard, QL standard, IQCS, and IQCL, the results indicating inhibited amplification of genomic DNA in the sample. As shown, some STR peaks are present, but the peaks show a "ski-slope" profile similar to the example of Figure 3D. unlike the example of Figure 3D, however, both the QL and the IQCL are reduced in height or absent in this situation. The absence of the IQCL indicates an amplification issue independent of low or degraded genomic DNA in the sample.

In some embodiments, the methods described herein include the steps of determining a first peak height (QS peak height) for a signal associated with the QS sequence and a second peak height (QL peak height) for a signal associated with the QL sequence, determining whether the QS peak height and/or QL peak height fall below respective predetermined thresholds, and flagging the sample as lower quality if the QS peak height and/or QL peak height fall below respective predetermined thresholds. The thresholds may be pre-set by a user, for example.

In some embodiments, when both the QS peak height and the QL peak height fall below their respective predetermined thresholds (e.g., such as in the example of Figure 3B), the sample is flagged for low nucleic acid concentration. In some embodiments, when the QS peak height and the QL peak height are both substantially zero (e.g., such as in the example of Figure 3C), the sample is flagged as lacking target nucleic acid.

In some embodiments, when the QS peak height is not lower than its threshold and the QL peak height is lower than its threshold (e.g., such as in the example of Figure 3D), the sample is flagged as having degraded target nucleic acid and/or as having inhibited amplification. In some embodiments, when the QS peak height is not lower than its threshold, the QL peak height is lower than its threshold, and the IQCL sequence is detected, the sample is flagged as having degraded target nucleic acid. In some embodiments, when the QS peak height is not lower than its threshold, the QL peak height is lower than its threshold, and the IQCL sequence is not detected, the sample is flagged as having inhibited amplification.

In some embodiments, the peak height of the QS standard, the peak heigh to the QL standard, or both are used to quantify an amount of genomic DNA in the sample. Because the QS and QL sequences from which the QS and QL standards are based are multi-copy sequences within the genomic DNA of the sample, the peak heights can be correlated to the quantity of DNA in the sample. This can be accomplished, for example, by generating a standard curve using known concentrations of DNA and subsequently utilizing the standard curve to calculate the quantity of sample DNA.

### Examples

### Example 1: Sample Processing Using a Rapid DNA Analysis Instrument

Sample processing was performed using a RapidHIT ID System (Applied Biosystems, Catalog No. A41810). A blood sample was collected using a flocked swab (HydraFlock Swab, available from Puritan Medical Products) and placed in the sample cartridge. The sample cartridge was pre-loaded with assay master mix, primers, and size standard to enable amplification and post-amplification analysis. The sample cartridge was then loaded into the instrument and a small volume (100 µL) lysis protocol was selected. The RapidHIT ID System then performed the sample lysis, DNA amplification, and capillary electrophoresis separation automatically in approximately 90 minutes.

The RapidHIT ID System automatically performed the following steps:
1) Lysis - lysate solution added and lysate mixture heated at 85° C.
2) Bubble Washing - lysate mixture agitated to mix and extract sample DNA from sample matrix.
3) Lysate Pull - lysis volume moved from sample collection chamber to the reaction chamber. A paper punch (1.5 mm diameter) absorbs a portion of the volume while the remaining lysate was flushed into waste chamber.
4) Premix Push - valves opened to allow master mix and primer mix to flood into reaction chamber and immerse the paper punch containing the sample lysate.
5) Thermal Cycling - PCR thermal cycling was set according to Table 3:

**Table 3: PCR Thermal Cycling**

| Phase | Step | Temperature | Duration (sec) |
|---|---|---|---|
| 1: Activation | Hold 1 | 95°C | 60 |
| | Hold 2 | 61.5°C | 30 |
| 2: Thermal Cycling (repeat 30 cycles) | Denature | 94°C | 3 |
| | Anneal/Extend | 61.5°C | 30 |
| 3: Final Hold | Hold | 60°C | 480 |

6) Diluent Push - the ILS size standard was mixed with water diluent and pushed into the reaction chamber to transfer the standard, diluent, and PCR reaction components to the mix chamber.
7) Air Pump - cartridge channel between reaction chamber and mix chamber was purged with air.
8) Sample Delivery - Denaturation: Sample mix (PCR reaction + size standard + diluent) passed through tubing in the Denature Heater (95°C) to denature amplified DNA products and size standard in preparation for CE. Sample DNA injected on CE (5,000 V for 40 kVs total).
9) Electrophoresis and Data Collection - heated CE capillary to 60°C, ramped up voltage to 9,000 V for approximately 25 minutes, collected fluorescent peak data, and transferred electronic data to files in a run folder on a network server.

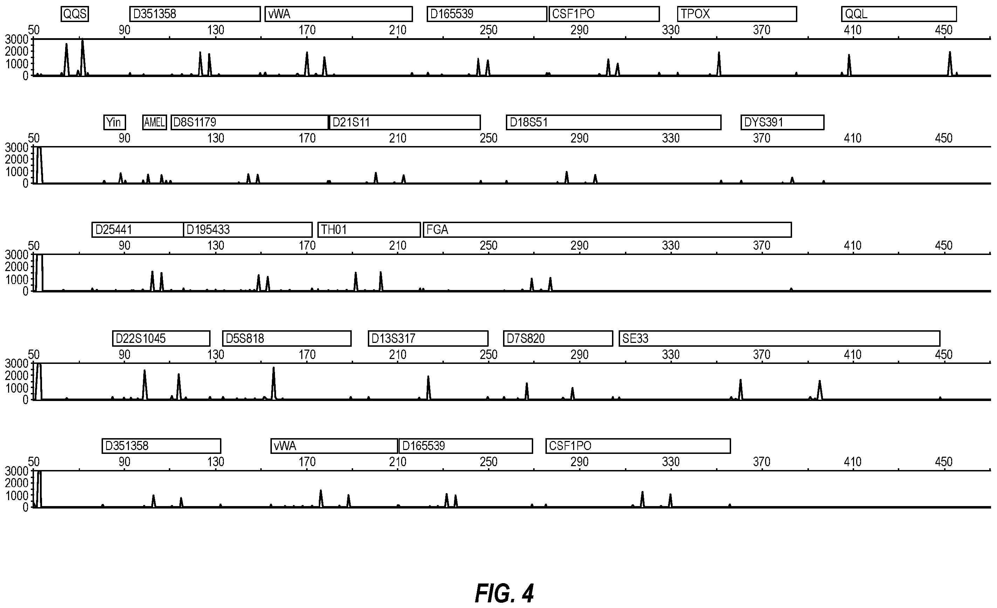

Figure 4 is a multi-channel electropherogram showing results of the above protocol using Control DNA 007 (see, e.g., Thermo Fisher Scientific, Catalog No. 100028107) and QS and QL markers selected from those shown in Figures 2A and 2B. Tested STR markers included D3S1358, vWA, D16S539, CSF1PO, TPOX, Y indel, AMEL, D8S1179, D21S11, D18S51, DYS391, D2S441, D19S433, TH01, FGA, D22S1045, D5S818, D13S317, D7S820, SE33, D10S1248, D1S1656, D12S391, and D2S1338, corresponding to the GlobalFiler^{™} amplification kit markers.

### Example 2: Blood Volume Peak Height Testing

Using the process as described in Example 1, a blood dilution series was tested to determine the effect of different blood volumes on resulting STR peak heights.

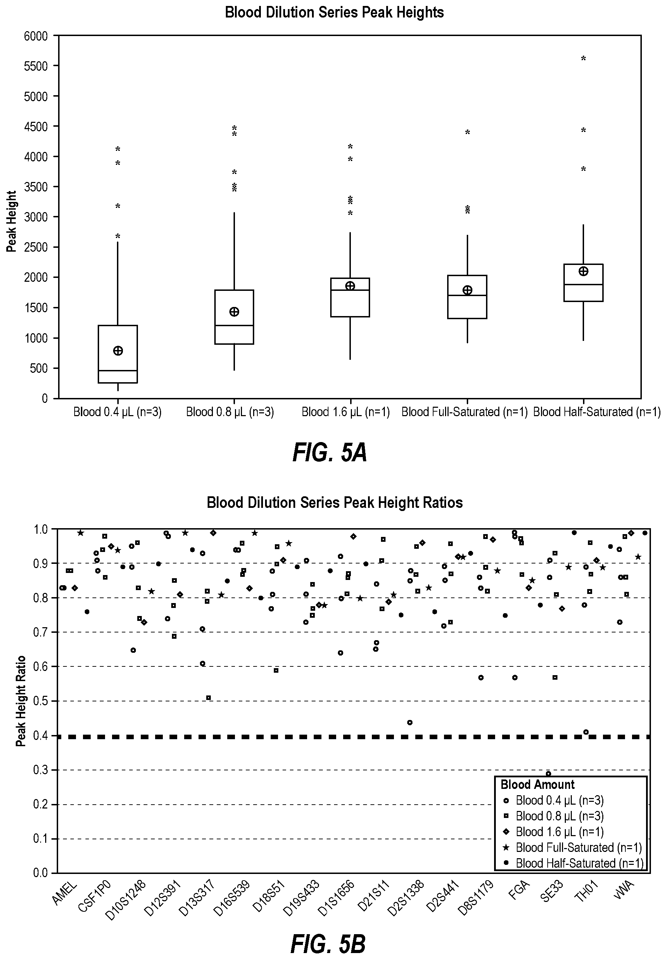

Figure 5A is a chart illustrating peak heights of STR profiles resulting from a blood dilution series that varied the volume of blood utilized in the rapid DNA analysis, the results showing that suitable peak heights were obtained at blood volumes as low as about 0.4 µL. Figure 5B is a chart illustrating peak height ratios for different blood volumes at various tested loci, the results showing suitable performance at blood volumes as low as about 0.4 µL.

Additional testing has shown effective results, with minimal allelic dropout, using blood volumes as low as 0.1 µL. Even with some allelic dropout at these low blood inputs, the profile balance is improved relative to conventional rapid investigative leads analysis methods. No allelic dropout was seen when testing blood volumes of 0.2 µL.

### Example 3: Comparison to Conventional Rapid Investigative Leads Analysis

Results from the process as described in Example 1 (1 µL blood input) were compared to results from a conventional rapid DNA process (1 µL blood using a standard cotton swab, 32 PCR cycles, 300 µL lysate).

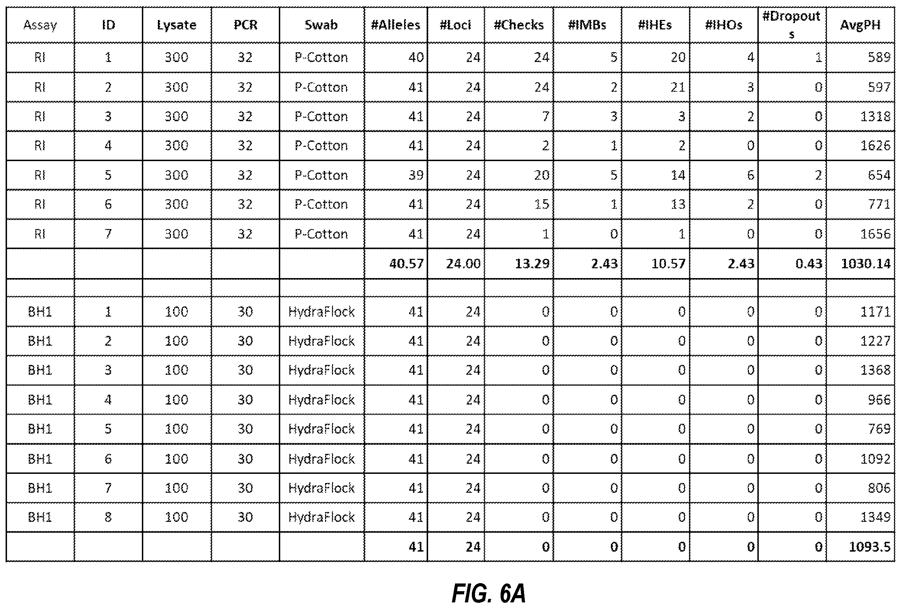

Figure 6A is a table comparing results of the conventional investigative leads process to an improved process according to the present disclosure, the improved process being modified to use a different collection swab, a lower lysate volume, and lower number of PCR cycles, showing that the modified process reduced the number of flags while also providing effective peak heights.

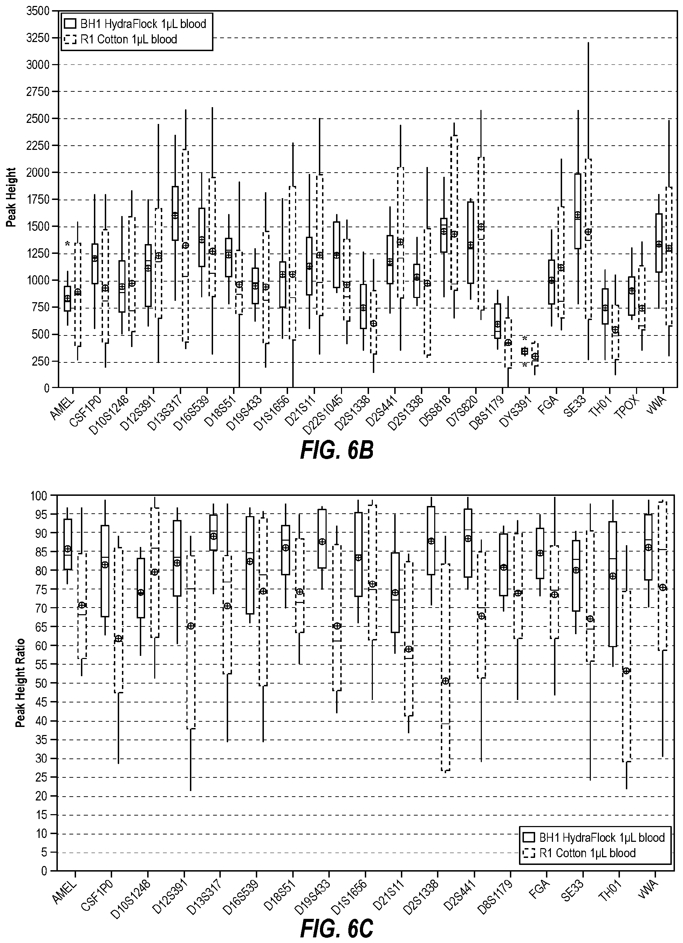

Figures 6B and 6C are charts illustrating peak heights (Figure 6B) and peak height ratios (Figure 6C) at various loci, comparing the conventional investigative leads process ("RI Cotton 1 µL blood") to an improved process according to the present disclosure ("BH1 HydraFlock 1 µL blood"), the results showing that the improved process led to less variance in peak heights.

### Example 4: DNA Input Variation Testing

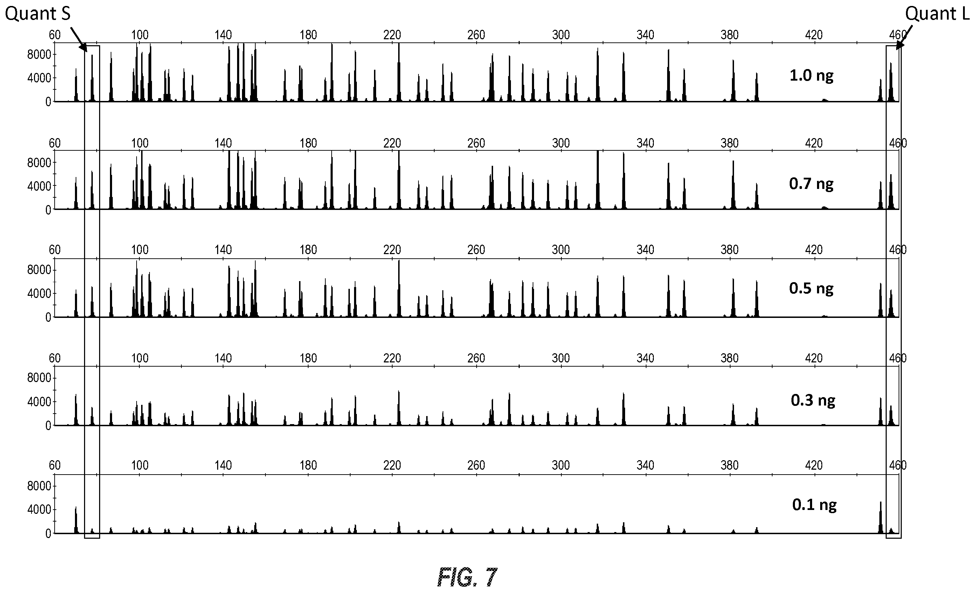

Using the process as described in Example 1 but with the inclusion of QS and QL primers, a series of different input DNA levels (1.0 ng, 0.7 ng, 0.5 ng, 0.3 ng, and 0.1 ng) were tested to determine the effects on QS and QL peaks. Figure 7 illustrates results of DNA input level testing, showing changes in peaks of the QS standard and QL standard in the corresponding STR profiles at different levels of input DNA. As shown, the QS and QL peak heights were proportional to the level of input DNA.

### Example 5: Quantification Marker Response

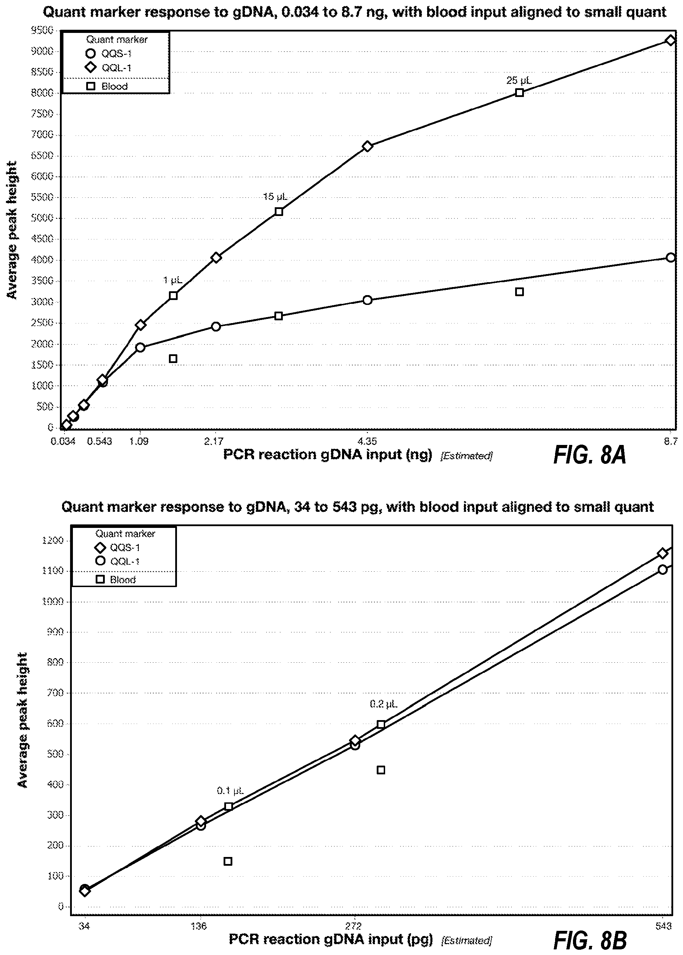

Average peak heights were measured across different levels of gDNA input based on Control DNA 007 (see, e.g., Thermo Fisher Scientific, Catalog No. 100028107) as well as liquid blood at 0.1 µL, 0.2 µL, 1 µL, 15 µL, and 25 µL. Results are shown in Figures 8A and 8B. Figure 8A shows the full set of data points (with input DNA shown in ng) and Figure 8B is an enlarged graph of the first four Control DNA 007 inputs (with input DNA shown in pg). The QS sequences are labelled as "QQS-1", and the QL sequences are labelled as "QQL-1." As shown, the peak heights for the blood samples were aligned with the QS curve, which matches well to the estimated PCR DNA capture for each blood volume. Although the QS and QL peak heights were somewhat different, the results are surprisingly effective considering they are roughly 340 base pairs apart from each other.

### Example 6: DNA Degradation Testing

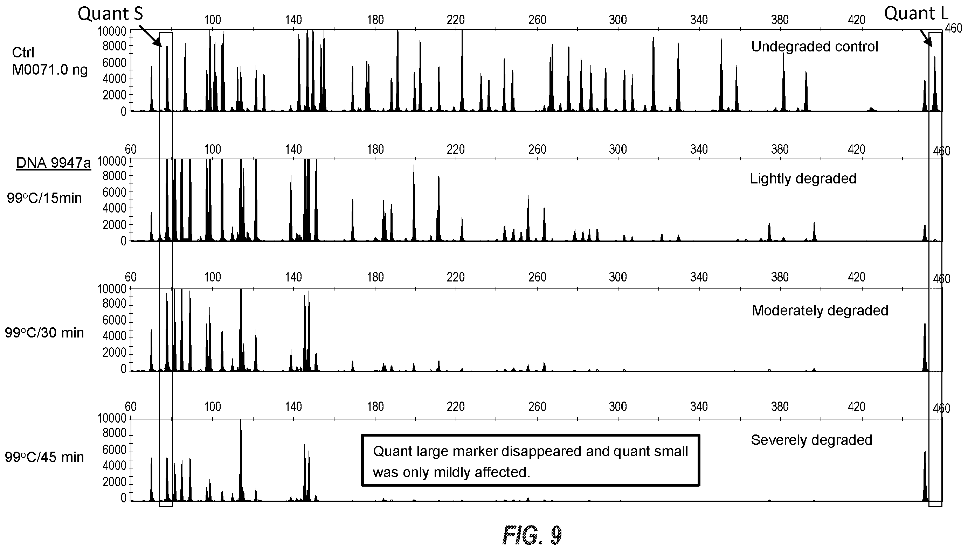

Using the process as described in Example 1 but with the inclusion of QS and QL primers, a series of progressively more degraded samples were tested. Figure 9 illustrates results of DNA degradation testing, showing greater changes to the QL standard than the QS standard in the corresponding STR profiles at progressively higher levels of DNA degradation.

### Additional Terms & Definitions

Note that this disclosure may alternatively refer to the QS standard as "QTS" or "QQS-QT", may alternatively refer to the QL standard as "QTL" or "QQL-QT", may alternatively refer to the IQCS as "QCS" or "QQS-QC", and may alternatively refer to the IQCL as "QCL" or "QQL-QC.

While certain embodiments of the present disclosure have been described in detail, with reference to specific configurations, parameters, components, elements, etcetera, the descriptions are illustrative and are not to be construed as limiting the scope of the claimed invention.

Furthermore, it should be understood that for any given element of component of a described embodiment, any of the possible alternatives listed for that element or component may generally be used individually or in combination with one another, unless implicitly or explicitly stated otherwise.

In addition, unless otherwise indicated, numbers expressing quantities, constituents, distances, or other measurements used in the specification and claims are to be understood as optionally being modified by the term "about" or its synonyms. When the terms "about," "approximately," "substantially," or the like are used in conjunction with a stated amount, value, or condition, it may be taken to mean an amount, value or condition that deviates by less than 20%, less than 10%, less than 5%, less than 1%, less than 0.1%, or less than 0.01% of the stated amount, value, or condition. At the very least, and not as an attempt to limit the application of the doctrine of equivalents to the scope of the claims, each numerical parameter should be construed in light of the number of reported significant digits and by applying ordinary rounding techniques.

It will also be noted that, as used in this specification and the appended claims, the singular forms "a," "an" and "the" do not exclude plural referents unless the context clearly dictates otherwise. Thus, for example, an embodiment referencing a singular referent (e.g., "widget") may also include two or more such referents.

## Claims

1. A method for amplifying one or more nucleic acids within a sample, the method comprising:
providing a sample that includes or is suspected of including nucleic acid;
forming a mixture by mixing with the sample a composition comprising:
at least one set of primers that target a short tandem repeat (STR) region of the sample nucleic acid,
a set of short quantification primers (QS primers) that target a first sequence (QS sequence) of the sample nucleic acid, and
a set of long quantification primers (QL primers) that target a second sequence (QL sequence) of the sample nucleic acid, wherein the QS sequence is shorter than the QL sequence; and
subjecting the mixture to amplification conditions to enable amplification of targeted nucleic acid if present within the sample.

2. The method of claim 1, further comprising identifying an allele of the STR region, or
wherein the composition comprises multiple sets of primers each configured to target a different STR region of the sample nucleic acid, optionally
further comprising identifying the alleles of the different STR regions, or
further comprising identifying an individual based on the determined allele(s), optionally wherein identifying the individual is associated with a forensic and/or crime scene investigation, optionally
wherein the QS sequence is shorter than the STR region(s) or
wherein the QL sequence is longer than the STR region(s).

3. The method of claim 1, wherein the sample is a forensic sample, crime scene sample, victim sample, or sample from unidentified human remains, or
wherein the sample comprises blood, bone, buccal material, saliva, semen, urine, feces, skin cells, touch DNA, or combination thereof, or
wherein the sample comprises human genomic DNA.

4. The method of claim 1, wherein the QS sequence, the QL sequence, or both are free of indels, or
wherein the QS sequence, the QL sequence, or both are multi-copy sequences.

5. The method of claim 1, wherein at least some of the forward primers and/or at least some of the reverse primers of the set of QS primers include a detectable label, optionally wherein the ratio of labelled to non-labelled forward primers in the set of QS primers is about 1:1 to about 1:5, such as about 1:2, and/or wherein the ratio of labelled to non-labelled reverse primers in the set of QS primers is about 1:1 to about 1:5, such as about 1:2.

6. The method of claim 1, wherein at least some of the forward primers and/or at least some of the reverse primers of the set of QL primers include a detectable label, optionally wherein the ratio of labelled to non-labelled forward primers in the set of QL primers is about 1:1 to about 1:5, such as about 1:2, and/or wherein the ratio of labelled to non-labelled reverse primers in the set of QL primers is about 1:1 to about 1:5, such as about 1:2.

7. The method of claim 1, wherein the set of QS primers is selected from SEQ ID NO:1 - SEQ ID NO:96 or
wherein the set of QL primers is selected from SEQ ID NO:97 - SEQ ID NO:192.

8. The method of claim 1, further comprising:
determining a first peak height (QS peak height) for a signal associated with the QS sequence and a second peak height (QL peak height) for a signal associated with the QL sequence;
determining whether the QS peak height and/or QL peak height fall below respective predetermined thresholds; and
flagging the sample as lower quality if the QS peak height and/or QL peak height fall below respective predetermined thresholds.

9. The method of claim 8, wherein when both the QS peak height and the QL peak height fall below their respective predetermined thresholds, the sample is flagged for low nucleic acid concentration, or
wherein when the QS peak height is not lower than its threshold and the QL peak height is lower than its threshold, the sample is flagged as having degraded target nucleic acid and/or as having inhibited amplification, or
wherein when the QS peak height and the QL peak height are both substantially zero, the sample is flagged as lacking target nucleic acid.

10. The method of claim 8, wherein the composition further comprises:
a short internal quality control (IQCS) comprising a synthetic IQCS sequence and a set of primers configured to enable amplification of the IQCS sequence; and
a long internal quality control (IQCL) comprising a synthetic IQCL sequence and a set of primers configured to enable amplification of the IQCL sequence,
wherein the IQCS sequence is shorter than the IQCL sequence.

11. The method of claim 10, wherein the IQCS sequence is shorter than the STR region(s), or
wherein the IQCL sequence is longer than the STR region(s), or
wherein when the QS peak height is not lower than its threshold, the QL peak height is lower than its threshold, and the IQCL sequence is detected, the sample is flagged as having degraded target nucleic acid, or
wherein when the QS peak height is not lower than its threshold, the QL peak height is lower than its threshold, and the IQCL sequence is not detected, the sample is flagged as having inhibited amplification.

12. The method of claim 1, wherein the composition further comprises a DNA polymerase and deoxyribonucleotide triphosphate (dNTP) molecules, and wherein subjecting the mixture to amplification conditions comprises performing a polymerase chain reaction (PCR).

13. The method of claim 12 , wherein the DNA polymerase is a thermostable DNA polymerase, optionally
wherein the DNA polymerase is Taq DNA polymerase, a mutant, variant, or derivative thereof.

14. The method of claim 12, wherein the nucleotides are selected from the group consisting of dTTP, dATP, dCTP, dGTP, 7-deaza-dGTP, or dUTP, or
the composition further comprising one or more buffers and one or more salts, or wherein the sample is collected using a non-cotton swab, or
wherein the sample is collected using a flocked swab, or
wherein the composition with which the sample is mixed further comprises a lysate, and wherein the composition has a volume of less than about 300 µl before mixing with the sample, or less than about 250 µl before mixing with the sample, or less than about 200 µl before mixing with the sample, or less than about 150 µl before mixing with the sample, or about 100 µl before mixing with the sample, or
wherein the PCR is performed with less than 32 cycles, such as 30 cycles, or
wherein the volume of sample mixed with the composition is at least about 0.1 µl and/or is no greater than about 35 µl, or
further comprising subjecting the amplified nucleic acid to a size separation process, or wherein the size separation process comprises capillary electrophoresis.

15. A composition formulated to enable analysis of nucleic acid samples, the composition comprising:
at least one set of primers that target a short tandem repeat (STR) region of the sample nucleic acid;
a set of short quantification primers that target a first sequence (QS sequence) of the sample nucleic acid; and
a set of long quantification primers that target a second sequence (QL sequence) of the sample nucleic acid,
wherein the QS sequence is shorter than the QL sequence.

16. The composition of claim 15, further comprising multiple sets of primers each configured to target a different STR region of the sample nucleic acid, or
wherein the QS sequence is shorter than the STR region(s), or
wherein the QL sequence is longer than the STR region(s), or
wherein the QS sequence, the QL sequence, or both are free of indels, or
wherein the QS sequence, the QL sequence, or both are multi-copy sequences.

17. The composition of claim 15, wherein at least some of the forward primers and/or at least some of the reverse primers of the set of QS primers include a detectable label, optionally
wherein the ratio of labelled to non-labelled forward primers in the set of QS primers is about 1:1 to about 1:5, such as about 1:2, and/or wherein the ratio of labelled to non-labelled reverse primers in the set of QS primers is about 1:1 to about 1:5, such as about 1:2, or
wherein at least some of the forward primers and/or at least some of the reverse primers of the set of QL primers include a detectable label, optionally
wherein the ratio of labelled to non-labelled forward primers in the set of QL primers is about 1:1 to about 1:5, such as about 1:2, and/or wherein the ratio of labelled to non-labelled reverse primers in the set of QL primers is about 1:1 to about 1:5, such as about 1:2, or
wherein the set of QS primers is selected from SEQ ID NO:1 - SEQ ID NO:96, or wherein the set of QL primers is selected from SEQ ID NO:97 - SEQ ID NO:192, or

18. The composition of claim 15, further comprising:
a short internal quality control (IQCS) comprising a synthetic IQCS sequence and a set of primers configured to enable amplification of the IQCS sequence; and
a long internal quality control (IQCL) comprising a synthetic IQCL sequence and a set of primers configured to enable amplification of the IQCL sequence,
wherein the IQCS sequence is shorter than the IQCL sequence.

19. The composition of claim 18, wherein the IQCS sequence is shorter than the STR region(s), or
wherein the IQCL sequence is longer than the STR region(s).

20. A kit for analysis of nucleic acid samples, the kit comprising:
the composition as in any one of claims 15-19;
a DNA polymerase; and
deoxyribonucleotide triphosphate (dNTP) molecules, optionally
wherein the DNA polymerase is a thermostable DNA polymerase. optionally
wherein the DNA polymerase is Taq DNA polymerase, a mutant, variant, or derivative thereof, or
wherein the dNTP molecules are selected from the group consisting of dTTP, dATP, dCTP, dGTP, 7-deaza-dGTP, or dUTP, or
further comprising one or more buffers and one or more salts.

## Patentansprüche

1. Verfahren zum Amplifizieren einer oder mehrerer Nukleinsäuren innerhalb einer Probe, das Verfahren umfassend:
Bereitstellen einer Probe, die Nukleinsäure einschließt oder von der vermutet wird, dass sie Nukleinsäure einschließt;
Ausbilden einer Mischung durch Mischen mit der Probe einer Zusammensetzung, umfassend:
mindestens einen Satz von Primern, die auf eine Mikrosatellitenregion (STR-Region) der Probennukleinsäure abzielen,
einen Satz von kurzen Quantifizierungsprimern (QS-Primer), die auf eine erste Sequenz (QS-Sequenz) der Probennukleinsäure abzielen, und
einen Satz von langen Quantifizierungsprimern (QL-Primer), die auf eine zweite Sequenz (QL-Sequenz) der Probennukleinsäure abzielen, wobei die QS-Sequenz kürzer als die QL-Sequenz ist; und
Aussetzen der Mischung gegenüber Amplifikationsbedingungen, um eine Amplifikation der gezielten Nukleinsäure zu ermöglichen, falls diese innerhalb der Probe vorhanden ist.

2. Verfahren nach Anspruch 1, ferner umfassend ein Identifizieren eines Allels der STR-Region,
oder
wobei die Zusammensetzung mehrere Sätze von Primern umfasst, die jeweils konfiguriert sind, um auf eine unterschiedliche STR-Region der Probennukleinsäure abzuzielen, optional
ferner umfassend das Identifizieren der Allele der verschiedenen STR-Regionen, oder
ferner umfassend das Identifizieren eines Individuums basierend auf dem/den bestimmten Allel(en), optional wobei das Identifizieren des Individuums mit einer forensischen und/oder Tatortuntersuchung assoziiert ist, optional
wobei die QS-Sequenz kürzer als die STR-Region(en) ist, oder
wobei die QL-Sequenz länger als die STR-Region(en) ist.

3. Verfahren nach Anspruch 1, wobei die Probe eine forensische Probe, eine Tatortprobe, eine Opferprobe oder eine Probe von nicht identifizierten menschlichen Überresten ist, oder
wobei die Probe Blut, Knochen, Wangenschleimhautmaterial, Speichel, Sperma, Urin, Fäzes, Hautzellen, Kontakt-DNA oder eine Kombination davon umfasst, oder
wobei die Probe menschliche genomische DNA umfasst.

4. Verfahren nach Anspruch 1, wobei die QS-Sequenz, die QL-Sequenz oder beide frei von Indels sind, oder
wobei die QS-Sequenz, die QL-Sequenz oder beide Mehrfachkopiesequenzen sind.

5. Verfahren nach Anspruch 1, wobei mindestens einige der Vorwärtsprimer und/oder mindestens einige der Rückwärtsprimer des Satzes von QS-Primern eine nachweisbare Markierung einschließen, optional wobei das Verhältnis von markierten zu nicht markierten Vorwärtsprimern in dem Satz von QS-Primern etwa 1 : 1 bis etwa 1 : 5 beträgt, wie etwa 1 : 2, und/oder wobei das Verhältnis von markierten zu nicht markierten Rückwärtsprimern in dem Satz von QS-Primern etwa 1 : 1 bis etwa 1 : 5 beträgt, wie etwa 1 : 2.

6. Verfahren nach Anspruch 1, wobei mindestens einige der Vorwärtsprimer und/oder mindestens einige der Rückwärtsprimer des Satzes von QL-Primern eine nachweisbare Markierung einschließen, optional wobei das Verhältnis von markierten zu nicht markierten Vorwärtsprimern in dem Satz von QL-Primern etwa 1 : 1 bis etwa 1 : 5 beträgt, wie etwa 1 : 2, und/oder wobei das Verhältnis von markierten zu nicht markierten Rückwärtsprimern in dem Satz von QL-Primern etwa 1 : 1 bis etwa 1 : 5 beträgt, wie etwa 1 : 2.

7. Verfahren nach Anspruch 1, wobei der Satz von QS-Primern aus SEQ ID NO: 1 - SEQ ID NO: 96 ausgewählt ist, oder
wobei der Satz von QL-Primern aus SEQ ID NO: 97 - SEQ ID NO: 192 ausgewählt ist.

8. Verfahren nach Anspruch 1, ferner umfassend:
Bestimmen einer ersten Peakhöhe (QS-Peakhöhe) für ein Signal, das mit der QS-Sequenz assoziiert ist, und einer zweiten Peakhöhe (QL-Peakhöhe) für ein Signal, das mit der QL-Sequenz assoziiert ist;
Bestimmen, ob die QS-Peakhöhe und/oder QL-Peakhöhe unter jeweilige zuvor bestimmte Schwellenwerte fallen; und
Kennzeichnen der Probe als von geringerer Qualität, falls die QS-Peakhöhe und/oder die QL-Peakhöhe unter entsprechende zuvor bestimmte Schwellenwerte fallen.

9. Verfahren nach Anspruch 8, wobei, wenn sowohl die QS-Peakhöhe als auch die QL-Peakhöhe unter ihre jeweiligen zuvor bestimmten Schwellenwerte fallen, die Probe wegen niedriger Nukleinsäurekonzentration gekennzeichnet wird, oder
wobei, wenn die QS-Peakhöhe nicht niedriger als ihr Schwellenwert ist und die QL-Peakhöhe niedriger als ihr Schwellenwert ist, die Probe als degradierte Zielnukleinsäure aufweisend und/oder als gehemmte Amplifikation aufweisend gekennzeichnet wird, oder
wobei, wenn die QS-Peakhöhe und die QL-Peakhöhe beide im Wesentlichen null sind, die Probe als fehlende Zielnukleinsäure gekennzeichnet wird.

10. Verfahren nach Anspruch 8, wobei die Zusammensetzung ferner umfasst:
eine kurze interne Qualitätskontrolle (IQCS), umfassend eine synthetische IQCS-Sequenz und einen Satz von Primern, die konfiguriert sind, um die Amplifikation der IQCS-Sequenz zu ermöglichen; und
eine lange interne Qualitätskontrolle (IQCL), umfassend eine synthetische IQCL-Sequenz und einen Satz von Primern, die konfiguriert sind, um die Amplifikation der IQCL-Sequenz zu ermöglichen,
wobei die IQCS-Sequenz kürzer als die IQCL-Sequenz ist.

11. Verfahren nach Anspruch 10, wobei die IQCS-Sequenz kürzer als die STR-Region(en) ist, oder
wobei die IQCL-Sequenz länger als die STR-Region(en) ist, oder
wobei, wenn die QS-Peakhöhe nicht niedriger als ihr Schwellenwert ist, die QL-Peakhöhe niedriger als ihr Schwellenwert ist und die IQCL-Sequenz detektiert wird, die Probe als eine degradierte Zielnukleinsäure aufweisend gekennzeichnet wird, oder
wobei, wenn die QS-Peakhöhe nicht niedriger als ihr Schwellenwert ist, die QL-Peakhöhe niedriger als ihr Schwellenwert ist und die IQCL-Sequenz nicht detektiert wird, die Probe als eine gehemmte Amplifikation aufweisend gekennzeichnet wird.

12. Verfahren nach Anspruch 1, wobei die Zusammensetzung ferner eine DNA-Polymerase und Desoxyribonukleotidtriphosphatmoleküle (dNTP-Moleküle) umfasst, und wobei
das Aussetzen der Mischung gegenüber Amplifikationsbedingungen das Durchführen einer Polymerasekettenreaktion (PCR) umfasst.

13. Verfahren nach Anspruch 12, wobei die DNA-Polymerase eine thermostabile DNA-Polymerase ist, gegebenenfalls
wobei die DNA-Polymerase Taq-DNA-Polymerase, eine Mutante, Variante oder ein Derivat davon ist.

14. Verfahren nach Anspruch 12, wobei die Nukleotide aus der Gruppe ausgewählt sind, bestehend aus dTTP, dATP, dCTP, dGTP, 7-Deaza-dGTP oder dUTP, oder
die Zusammensetzung ferner umfassend einen oder mehrere Puffer und ein oder mehrere Salze, oder wobei die Probe unter Verwendung eines Nichtbaumwolltupfers gesammelt wird, oder
wobei die Probe unter Verwendung eines beflockten Tupfers gesammelt wird, oder
wobei die Zusammensetzung, mit der die Probe gemischt wird, ferner ein Lysat umfasst, und wobei die Zusammensetzung ein Volumen von weniger als etwa 300 µl vor dem Mischen mit der Probe oder weniger als etwa 250 µl vor dem Mischen mit der Probe oder weniger als etwa 200 µl vor dem Mischen mit der Probe oder weniger als etwa 150 µl vor dem Mischen mit der Probe oder etwa 100 µl vor dem Mischen mit der Probe aufweist, oder
wobei die PCR mit weniger als 32 Zyklen durchgeführt wird, wie 30 Zyklen, oder
wobei das Volumen der mit der Zusammensetzung gemischten Probe mindestens etwa 0,1 µl beträgt und/oder nicht größer als etwa 35 µl ist, oder
ferner umfassend das Aussetzen der amplifizierten Nukleinsäure gegenüber einem Größentrennungsprozess oder wobei der Größentrennungsprozess Kapillarelektrophorese umfasst.

15. Zusammensetzung, die formuliert ist, um eine Analyse von Nukleinsäureproben zu ermöglichen, die Zusammensetzung umfassend:
mindestens ein Satz von Primern, die auf eine Mikrosatellitenregion (STR-Region) der Probennukleinsäure abzielen;
ein Satz von kurzen Quantifizierungsprimern, die auf eine erste Sequenz (QS-Sequenz) der Probennukleinsäure abzielen; und
einen Satz langer Quantifizierungsprimer, die auf eine zweite Sequenz (QL-Sequenz) der Probennukleinsäure abzielen,
wobei die QS-Sequenz kürzer als die QL-Sequenz ist.

16. Zusammensetzung nach Anspruch 15, ferner umfassend mehrere Sätze von Primern, die jeweils konfiguriert sind, um auf eine unterschiedliche STR-Region der Probennukleinsäure abzuzielen, oder
wobei die QS-Sequenz kürzer als die STR-Region(en) ist, oder
wobei die QL-Sequenz länger als die STR-Region(en) ist, oder
wobei die QS-Sequenz, die QL-Sequenz oder beide frei von Indels sind, oder
wobei die QS-Sequenz, die QL-Sequenz oder beide Mehrfachkopiesequenzen sind.

17. Zusammensetzung nach Anspruch 15, wobei mindestens einige der Vorwärtsprimer und/oder mindestens einige der Rückwärtsprimer des Satzes von QS-Primern eine nachweisbare Markierung einschließen, optional
wobei das Verhältnis von markierten zu nicht markierten Vorwärtsprimern in dem Satz von QS-Primern etwa 1 : 1 bis etwa 1 : 5 beträgt, wie etwa 1 : 2, und/oder wobei das Verhältnis von markierten zu nicht markierten Rückwärtsprimern in dem Satz von QS-Primern etwa 1 : 1 bis etwa 1 : 5 beträgt, wie etwa 1 : 2, oder
wobei mindestens einige der Vorwärtsprimer und/oder mindestens einige der Rückwärtsprimer des Satzes von QL-Primern eine nachweisbare Markierung einschließen, optional
wobei das Verhältnis von markierten zu nicht markierten Vorwärtsprimern in dem Satz von QL-Primern etwa 1 : 1 bis etwa 1 : 5 beträgt, wie etwa 1 : 2, und/oder wobei das Verhältnis von markierten zu nicht markierten Rückwärtsprimern in dem Satz von QL-Primern etwa 1 : 1 bis etwa 1 : 5 beträgt, wie etwa 1 : 2, oder
wobei der Satz von QS-Primern aus SEQ ID NO: 1 - SEQ ID NO: 96 ausgewählt ist, oder wobei der Satz von QL-Primern aus SEQ ID NO: 97 - SEQ ID NO: 192 ausgewählt ist, oder

18. Zusammensetzung nach Anspruch 15, ferner umfassend:
eine kurze interne Qualitätskontrolle (IQCS), umfassend eine synthetische IQCS-Sequenz und einen Satz von Primern, die konfiguriert sind, um die Amplifikation der IQCS-Sequenz zu ermöglichen; und
eine lange interne Qualitätskontrolle (IQCL), umfassend eine synthetische IQCL-Sequenz und einen Satz von Primern, die konfiguriert sind, um die Amplifikation der IQCL-Sequenz zu ermöglichen,
wobei die IQCS-Sequenz kürzer als die IQCL-Sequenz ist.

19. Zusammensetzung nach Anspruch 18, wobei die IQCS-Sequenz kürzer als die STR-Region(en) ist, oder
wobei die IQCL-Sequenz länger als die STR-Region(en) ist.

20. Kit zum Analysieren von Nukleinsäureproben, das Kit umfassend:
die Zusammensetzung nach einem der Ansprüche 15 bis 19;
eine DNA-Polymerase; und
Desoxyribonukleotidtriphosphatmoleküle (dNTP-Moleküle), optional
wobei die DNA-Polymerase eine thermostabile DNA-Polymerase ist, optional
wobei die DNA-Polymerase Taq-DNA-Polymerase, eine Mutante, Variante oder ein Derivat davon ist, oder
wobei die dNTP-Moleküle aus der Gruppe ausgewählt sind, bestehend aus dTTP, dATP, dCTP, dGTP, 7-Deaza-dGTP oder dUTP, oder
ferner umfassend einen oder mehrere Puffer und ein oder mehrere Salze.

## Revendications

1. Procédé d'amplification d'un ou plusieurs acides nucléiques dans un échantillon, le procédé comprenant :
la fourniture d'un échantillon qui comporte ou est soupçonné de comporter un acide nucléique ;
la formation d'un mélange en mélangeant avec l'échantillon une composition comprenant :
au moins un ensemble d'amorces qui ciblent une région de courtes répétitions en tandem (STR) de l'acide nucléique d'échantillon,
un ensemble d'amorces de quantification courtes (amorces QS) qui ciblent une première séquence (séquence QS) de l'acide nucléique d'échantillon, et
un ensemble d'amorces de quantification longues (amorces QL) qui ciblent une seconde séquence (séquence QL) de l'acide nucléique d'échantillon, dans lequel la séquence QS est plus courte que la séquence QL ; et
le fait de soumettre le mélange à des conditions d'amplification afin de permettre une amplification d'acide nucléique ciblé s'il est présent dans l'échantillon.

2. Procédé selon la revendication 1, comprenant en outre l'identification d'un allèle de la région STR, ou
dans lequel la composition comprend de multiples ensembles d'amorces, chacune étant conçue pour cibler une région STR différente de l'acide nucléique d'échantillon, facultativement
comprenant en outre l'identification des allèles des différentes régions STR, ou
comprenant en outre l'identification d'un individu sur la base du ou des allèles déterminés, facultativement dans lequel l'identification de l'individu est associée à une enquête médico-légale et/ou sur une scène de crime, facultativement
dans lequel la séquence QS est plus courte que la ou les régions STR ou
dans lequel la séquence QL est plus longue que la ou les régions STR.

3. Procédé selon la revendication 1, dans lequel l'échantillon est un échantillon médico-légal, un échantillon de scène de crime, un échantillon de victime, ou un échantillon provenant de restes humains non identifiés, ou
dans lequel l'échantillon comprend du sang, des os, du matériel buccal, de la salive, du sperme, de l'urine, des fèces, des cellules cutanées, de l'ADN de contact, ou une combinaison de ceux-ci, ou
dans lequel l'échantillon comprend de l'ADN génomique humain.

4. Procédé selon la revendication 1, dans lequel la séquence QS, la séquence QL, ou les deux, sont exemptes d'indels, ou
dans lequel la séquence QS, la séquence QL, ou les deux, sont des séquences multi-copies.

5. Procédé selon la revendication 1, dans lequel au moins une partie des amorces sens et/ou au moins une partie des amorces antisens de l'ensemble d'amorces QS comportent un marqueur détectable, facultativement dans lequel le rapport entre les amorces sens marquées et non marquées dans l'ensemble d'amorces QS est d'environ 1:1 à environ 1:5, par exemple d'environ 1:2, et/ou dans lequel le rapport entre les amorces antisens marquées et non marquées dans l'ensemble d'amorces QS est d'environ 1:1 à environ 1:5, par exemple d'environ 1:2.

6. Procédé selon la revendication 1, dans lequel au moins une partie des amorces sens et/ou au moins une partie des amorces antisens de l'ensemble d'amorces QL comportent un marqueur détectable, facultativement dans lequel le rapport entre les amorces sens marquées et non marquées dans l'ensemble d'amorces QL est d'environ 1:1 à environ 1:5, par exemple d'environ 1:2, et/ou dans lequel le rapport entre les amorces antisens marquées et non marquées dans l'ensemble d'amorces QL est d'environ 1:1 à environ 1:5, par exemple d'environ 1:2.

7. Procédé selon la revendication 1, dans lequel l'ensemble d'amorces QS est choisi parmi SEQ ID NO:1 à SEQ ID NO:96 ou
dans lequel l'ensemble d'amorces QL est choisi parmi SEQ ID NO:97 à SEQ ID NO: 192.

8. Procédé selon la revendication 1, comprenant en outre :
la détermination d'une première hauteur de pic (hauteur de pic QS) pour un signal associé à la séquence QS et une seconde hauteur de pic (hauteur de pic QL) pour un signal associé à la séquence QL ;
le fait de déterminer si la hauteur de pic QS et/ou la hauteur de pic QL sont inférieures à des seuils prédéterminés respectifs ; et
le signalement de l'échantillon comme étant de qualité inférieure si la hauteur de pic QS et/ou la hauteur de pic QL sont inférieures à des seuils prédéterminés respectifs.

9. Procédé selon la revendication 8, dans lequel, lorsque la hauteur de pic QS et que la hauteur de pic QL sont toutes deux inférieures à leurs seuils prédéterminés respectifs, l'échantillon est signalé comme ayant une faible concentration en acide nucléique, ou
dans lequel, lorsque la hauteur de pic QS n'est pas inférieure à son seuil et que la hauteur de pic QL est inférieure à son seuil, l'échantillon est signalé comme ayant un acide nucléique cible dégradé et/ou comme ayant une amplification inhibée, ou
dans lequel, lorsque la hauteur de pic QS et que la hauteur de pic QL sont toutes deux sensiblement nulles, l'échantillon est signalé comme étant dépourvu d'acide nucléique cible.

10. Procédé selon la revendication 8, dans lequel la composition comprend en outre :
un contrôle de qualité interne court (IQCS) comprenant une séquence synthétique IQCS et un ensemble d'amorces conçues pour permettre une amplification de la séquence IQCS ; et
un contrôle de qualité interne long (IQCL) comprenant une séquence synthétique IQCL et un ensemble d'amorces conçues pour permettre une amplification de la séquence IQCL,
dans lequel la séquence IQCS est plus courte que la séquence IQCL.

11. Procédé selon la revendication 10, dans lequel la séquence IQCS est plus courte que la ou les régions STR, ou
dans lequel la séquence IQCL est plus longue que la ou les régions STR, ou
dans lequel, lorsque la hauteur de pic QS n'est pas inférieure à son seuil, que la hauteur de pic QL est inférieure à son seuil, et que la séquence IQCL est détectée, l'échantillon est signalé comme ayant un acide nucléique cible dégradé, ou
dans lequel, lorsque la hauteur de pic QS n'est pas inférieure à son seuil, que la hauteur de pic QL est inférieure à son seuil, et que la séquence IQCL n'est pas détectée, l'échantillon est signalé comme ayant une amplification inhibée.

12. Procédé selon la revendication 1, dans lequel la composition comprend en outre une ADN polymérase et des molécules de désoxyribonucléotide triphosphate (dNTP), et dans lequel
le fait de soumettre le mélange à des conditions d'amplification comprend la réalisation d'une réaction en chaîne par polymérase (PCR).

13. Procédé selon la revendication 12, dans lequel l'ADN polymérase est une ADN polymérase thermostable, facultativement
dans lequel l'ADN polymérase est la Taq ADN polymérase, un mutant, un variant, ou un dérivé de celle-ci.

14. Procédé selon la revendication 12, dans lequel les nucléotides sont choisis dans le groupe constitué de dTTP, dATP, dCTP, dGTP, 7-déaza-dGTP, ou dUTP, ou
la composition comprenant en outre un ou plusieurs tampons et un ou plusieurs sels, ou dans lequel l'échantillon est prélevé à l'aide d'un écouvillon autre qu'un coton, ou
dans lequel l'échantillon est prélevé à l'aide d'un écouvillon floqué, ou
dans lequel la composition avec laquelle l'échantillon est mélangé comprend en outre un lysat, et dans lequel la composition a un volume inférieur à environ 300 µl avant le mélange avec l'échantillon, ou inférieur à environ 250 µl avant le mélange avec l'échantillon, ou inférieur à environ 200 µl avant le mélange avec l'échantillon, ou inférieur à environ 150 µl avant le mélange avec l'échantillon, ou d'environ 100 µl avant le mélange avec l'échantillon, ou
dans lequel la PCR est effectuée avec moins de 32 cycles, par exemple 30 cycles, ou
dans lequel le volume d'échantillon mélangé avec la composition est d'au moins environ 0,1 µl et/ou n'est pas supérieur à environ 35 µl, ou
comprenant en outre le fait de soumettre l'acide nucléique amplifié à un processus de séparation de taille, ou dans lequel le processus de séparation de taille comprend une électrophorèse capillaire.

15. Composition formulée dans le but de permettre une analyse d'échantillons d'acides nucléiques, la composition comprenant :
au moins un ensemble d'amorces qui ciblent une région de courtes répétitions en tandem (STR) de l'acide nucléique d'échantillon ;
un ensemble d'amorces de quantification courtes qui ciblent une première séquence (séquence QS) de l'acide nucléique d'échantillon ; et
un ensemble d'amorces de quantification longues qui ciblent une seconde séquence (séquence QL) de l'acide nucléique d'échantillon,
dans laquelle la séquence QS est plus courte que la séquence QL.

16. Composition selon la revendication 15, comprenant en outre de multiples ensembles d'amorces chacune étant conçue pour cibler une région STR différente de l'acide nucléique d'échantillon, ou
dans laquelle la séquence QS est plus courte que la ou les régions STR, ou
dans laquelle la séquence QL est plus longue que la ou les régions STR, ou
dans laquelle la séquence QS, la séquence QL, ou les deux, sont exemptes d'indels, ou
dans laquelle la séquence QS, la séquence QL, ou les deux, sont des séquences multi-copies.

17. Composition selon la revendication 15, dans laquelle au moins une partie des amorces sens et/ou au moins une partie des amorces antisens de l'ensemble d'amorces QS comportent un marqueur détectable, facultativement
dans laquelle le rapport entre les amorces sens marquées et non marquées dans l'ensemble d'amorces QS est d'environ 1:1 à environ 1:5, par exemple d'environ 1:2, et/ou dans laquelle le rapport entre les amorces antisens marquées et non marquées dans l'ensemble d'amorces QS est d'environ 1:1 à environ 1:5, par exemple d'environ 1:2, ou
dans laquelle au moins une partie des amorces sens et/ou au moins une partie des amorces antisens de l'ensemble d'amorces QL comportent un marqueur détectable, facultativement
dans laquelle le rapport entre les amorces sens marquées et non marquées dans l'ensemble d'amorces QL est d'environ 1:1 à environ 1:5, par exemple d'environ 1:2, et/ou dans laquelle le rapport entre les amorces antisens marquées et non marquées dans l'ensemble d'amorces QL est d'environ 1:1 à environ 1:5, par exemple d'environ 1:2, ou
dans laquelle l'ensemble d'amorces QS est choisi parmi SEQ ID NO: 1 à SEQ ID NO:96, ou dans laquelle l'ensemble d'amorces QL est choisi parmi SEQ ID NO:97 à SEQ ID NO: 192, ou

18. Composition selon la revendication 15, comprenant en outre :
un contrôle de qualité interne court (IQCS) comprenant une séquence synthétique IQCS et un ensemble d'amorces conçues pour permettre une amplification de la séquence IQCS ; et
un contrôle de qualité interne long (IQCL) comprenant une séquence synthétique IQCL et un ensemble d'amorces conçues pour permettre une amplification de la séquence IQCL,
dans laquelle la séquence IQCS est plus courte que la séquence IQCL.

19. Composition selon la revendication 18, dans laquelle la séquence IQCS est plus courte que la ou les régions STR, ou
dans laquelle la séquence IQCL est plus longue que la ou les régions STR.

20. Trousse pour l'analyse d'échantillons d'acides nucléiques, la trousse comprenant :
la composition selon l'une quelconque des revendications 15 à 19 ;
une ADN polymérase ; et
des molécules de désoxyribonucléotide triphosphate (dNTP), facultativement
dans laquelle l'ADN polymérase est une ADN polymérase thermostable, facultativement
dans laquelle l'ADN polymérase est la Taq ADN polymérase, un mutant, un variant, ou un dérivé de celle-ci, ou
dans laquelle les molécules dNTP sont choisies dans le groupe constitué de dTTP, dATP, dCTP, dGTP, 7-déaza-dGTP, ou dUTP, ou
comprenant en outre un ou plusieurs tampons et un ou plusieurs sels.
